# EUROPEAN PATENT SPECIFICATION

(11) **EP 3 061 443 B1**
(45) Date of publication and mention of the grant of the patent: **14.08.2019**
(21) Application number: 15382076.6
(22) Date of filing: 24.02.2015
(51) Int. Cl.: A61K 8/73, A61Q 19/06, A61Q 19/08, A61K 8/92, A61K 8/97, A61K 8/04

(54) **A process for the cosmetic treatment of skin**
Verfahren zur kosmetischen Behandlung der Haut
Procédé de traitement cosmétique de la peau

(43) Date of publication of application: 31.08.2016
(73) Proprietor: Tecnologia Para la Medicina y la Estetica S.L., 08028 Barcelona (ES)
(72) Inventor: Bertrán Segarra, Francisco, 08021 BARCELONA (ES); Del Río Pin, Maria Otilia, 08021 BARCELONA (ES)
(74) Representative: Ponti & Partners, S.L.P

(56) References cited:
- US-A1- 2011 144 565
- US-B- 8 467 868
- Robert A Weiss ET AL: "Noninvasive radio frequency for skin tightening and body contouring", Seminars in cutaneous medicine and surgery, 1 March 2013 (2013-03-01), page 9, XP055204680, United States Retrieved from the Internet: URL:http://www.ncbi.nlm.nih.gov/pubmed/240 49924 [retrieved on 2015-07-27]
- MARIO A TRELLES ET AL: "Histological findings in adipocytes when cellulite is treated with a variable-emission radiofrequency system", LASERS IN MEDICAL SCIENCE, SPRINGER-VERLAG, LO, vol. 25, no. 2, 26 March 2009 (2009-03-26) , pages 191-195, XP019763724, ISSN: 1435-604X
- Anonymous: "How to choose the right radiofrequency treatment for skin tightening (face/body) and cellulite reduction - LipoTherapeia - Most advanced cellulite treatments in London", , 18 July 2012 (2012-07-18), XP055204714, Retrieved from the Internet: URL:http://www.lipotherapeia.com/the-peach -factor-blog/what-determines-the-effective ness-of-a-radiofrequency-treatment [retrieved on 2015-07-27]
- "The difference is INDIBA", , 12 July 2013 (2013-07-12), XP055204808, Retrieved from the Internet: URL:http://www.indibasia.com/pdf/Indiba 12ppSupplement FA.pdf [retrieved on 2015-07-27]
- "Scientific literature", , 12 December 2012 (2012-12-12), XP055204933, Retrieved from the Internet: URL:http://www.indiba-germany.de/fileadmin /tmpl/files/scientific_literature.pdf [retrieved on 2015-07-28]

## Description

### FIELD OF THE INVENTION

The present invention relates to a process for the cosmetic treatment of the skin comprising the application of diathermy with a conductive gel and oil and/or certain other substances.

### BACKGROUND OF THE INVENTION

Since the last century hyperthermy or also called diathermy devices are known, mainly used in rehabilitation, whose main characteristic is the application of heat internally in the body without changing the temperature of the skin on the outer part or epidermis.

In these devices, if the current is transmitted capacitively (short wave), the diathermy is called capacitive diathermy and has applications in the field of aesthetics, for example in the treatment of wrinkles, sagging skin, stretch marks, cellulite, eye bags, transepidermal improved penetration of cosmetic products, etc ... For its application metallic electrodes are arranged in direct contact with the skin through a conductive gel. The return electrode is placed as close as possible to the area to be treated. The emission mode (which can be pulsed or continuous) will be pulsed as it decreases the subjective sensation of heat in the area, allowing to obtain the same effects. It is indicated for heat sensitive people. The electrode should slide through the area to be treated, without keeping it fixed at a point and without touching the person during the treatment time, since a great increase of heat in the touched area would be felt.

Diathermy is applicable in capacitive or resistive mode. The capacitive method increases the temperature more rapidly in the epidermal part (surface), but less energy penetrates internally than with resistive method.

When applying diathermy a spark or electric arc between the electrode and the skin typically occurs, which may be harmful to the subject depending on the applied intensity.

Surprisingly, the present inventors have devised a new process in which aesthetic problems, such as wrinkles, sagging, stretch marks, cellulite, eye bags, etc, are improved/solved, by applying diathermy in combination with the application of a conductive gel and oils and/or other substances with the added possibility of applying cosmetic active ingredients and without causing the spark or electric arc typical for diathermy.

### BRIEF DESCRIPTION OF DRAWINGS

Figure 1 shows a series of three photographs showing the progress of cellulite before and after application of the process of the invention.
Figures 2 (a) and 2 (b) show pairs of photographs showing the reduction of wrinkles after application of the process of the invention.

### DESCRIPTION OF THE INVENTION

The present invention relates to a process for the cosmetic treatment of the skin comprising applying to the affected skin diathermy along with a conductive gel and oil(s) and/or another substance selected from the group consisting of hyaluronic acid, shea butter, aloe vera and vitamins, or a combination thereof (i.e., any possible combination among hyaluronic acid, shea butter, aloe vera and vitamins), wherein the conductive gel is applied with diathermy in resistive mode, and the oil or oils are applied with diathermy in capacitive mode.

As noted above, the capacitive diathermy is used to heat the skin at surface level. To perform diathermy a device comprising a control unit and a unit generating electrical signals which are sent to terminals, intended to be applied to the skin is used. The terminals have a contact plate with the skin and can be resistive or capacitive. The terminals are mounted on ergonomic pressure handles. Signals operate at high frequencies, typically between 0.5 MHz and 0.8 MHz. The power is usually 300/220 W.

In the context of the present invention a "conductive gel" is a conductive moisturizing composition used for diathermy devices. This gel allows the proper movement of the electrodes and the correct transmission of electric current. Its use will prevent spikes in temperature, having a cooling effect of temperature, facilitating the hydration of the areas to be treated and increasing the system efficiency. This conductive gel can also incorporate cosmetic active principles.

In a preferred embodiment, the oil used in the process of the present invention is selected from almond oil, argan oil, horse-chestnut oil, eucalyptus oil, natural wintergreen oil, rosehip oil, carrot oil, camphor oil, firming oils, anti-cellulite oils, and lipolytic oils, or a combination thereof. More preferably the oil is almond oil, argan oil, horse-chestnut oil, eucalyptus oil, natural wintergreen oil, rosehip oil, carrot oil, camphor oil, or a combination thereof.

Diathermy will be applied for a certain time depending on the specific treatment. In a preferred embodiment, diathermy is applied between 20 minutes and 1 hour and half daily. In a more preferred embodiment, diathermy will be applied half the time in capacitive mode and half the time in resistive mode. In a more preferred embodiment, the conductive gel is applied with diathermy in resistive mode; and oil(s) and/or other substance(s) selected from the group consisting of hyaluronic acid, shea butter, aloe vera and vitamins, or a combination thereof, will be applied with diathermy in capacitive mode.

In addition to remove the spark or electric arc, the present treatment achieves two main goals:
- on the one hand, the permeability of the membrane of the epidermis is increased, allowing the correct insertion of cosmetic active ingredients; and
- on the other hand, the thermal effect will cause a local hyperthermy which allow a better use of the cosmetic active ingredient in tissue and therefore a more aesthetic effect.

The present invention is now illustrated with examples that are not intended to limit the scope thereof.

### EXAMPLE

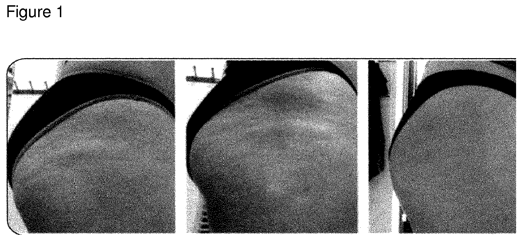

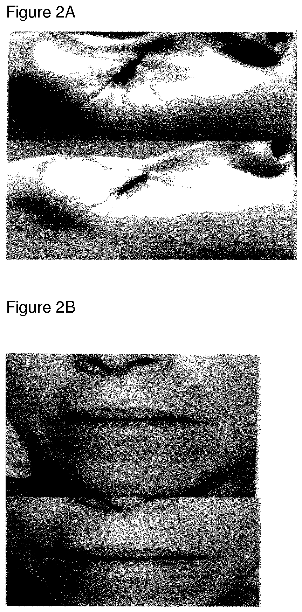

By way of example the process of the invention is carried out by applying resistive diathermy for 10 minutes in combination with the application of a conductive gel onto the skin. After these 10 minutes diathermy is applied in capacitive mode for another 10 minutes in combination with the application of oils and/or other substances selected from the group consisting of hyaluronic acid, shea butter, aloe vera and vitamins, or a combination thereof. The treatment is repeated for two nonconsecutive days, although depending on the type of treatment and the severity of the problem it can be performed more than two sessions. Figures 1 and 2A, 2B show the results before and after application of the process of the invention for treating cellulite and wrinkles around the eyes.

## Claims

1. Process for the cosmetic treatment of the skin comprising applying to the affected skin diathermy along with a conductive gel and one or more oils and/or another substance selected from the group consisting of hyaluronic acid, shea butter, aloe vera and vitamins, or a combination thereof, wherein the conductive gel is applied with diathermy in resistive mode and the oil or oils are applied with diathermy in capacitive mode.

2. Process according to claim 1, wherein said oil is selected from the group consisting of almond oil, argan oil, horse-chestnut oil, eucalyptus oil, natural wintergreen oil, rosehip oil, carrot oil, camphor oil, firming oils, anti-cellulite oils, and lipolytic oils, or a combination thereof.

3. Process according to claim 2, wherein said oil is almond oil, argan oil, horse-chestnut oil, eucalyptus oil, natural wintergreen oil, rosehip oil, carrot oil, camphor oil, or a combination thereof.

4. Process according to any of the preceding claims, wherein diathermy is applied between 20 minutes and 1 hour and half daily.

5. Process according to any of the preceding claims, wherein diathermy is applied half the time in capacitive mode and half the time in resistive mode.

6. Process according to any of the preceding claims, wherein said other substance selected from the group consisting of hyaluronic acid, shea butter, aloe vera and vitamins, or a combination thereof, is applied with diathermy in capacitive mode.

7. Process according to any of the preceding claims, wherein the diathermy is applied at a frequency between 0.5 MHz and 0.8 MHz.

## Patentansprüche

1. Verfahren zur kosmetischen Behandlung der Haut umfassend das Applizieren von Diathermie auf die betroffene Haut zusammen mit einem leitfähigem Gel und einem oder mehreren Ölen und/oder weiterer Substanz, die aus der Gruppe, die aus Hyaluronsäure, Sheabutter, Aloe Vera und Vitaminen, oder einer Kombination davon besteht, ausgewählt wird, wobei das leitfähige Gel mit Diathermie in resistiver Betriebsweise appliziert wird und das Öl oder die Öle mit Diathermie in kapazitiver Betriebsweise appliziert werden.

2. Verfahren nach Anspruch 1, wobei das Öl aus der Gruppe, die aus Mandelöl, Arganöl, Rosskastanienöl, Eukalyptusöl, natürlichem Wintergrünöl, Hagebuttenöl, Karottenöl, Kampferöl, straffenden Ölen, Anti-Cellulite-Ölen, und lipolitischen Ölen, oder einer Kombination davon besteht, ausgewählt wird.

3. Verfahren nach Anspruch 2, wobei das Öl Mandelöl, Arganöl, Rosskastanienöl, Eukalyptusöl, natürliches Wintergrünöl, Hagebuttenöl, Karottenöl, Kampferöl oder eine Kombination davon ist.

4. Verfahren nach einem der vorhergehenden Ansprüche, wobei Diathermie zwischen 20 Minuten und einer Stunde und halbtäglich appliziert wird.

5. Verfahren nach einem der vorhergehenden Ansprüche, wobei Diathermie zur Hälfte der Zeit in kapazitiver Betriebsweise und zur Hälfte der Zeit in resistiver Betriebsweise appliziert wird.

6. Verfahren nach einem der vorhergehenden Ansprüche, wobei die weitere Substanz aus der Gruppe, die aus Hyaluronsäure, Sheabutter, Aloe Vera und Vitaminen, oder einer Kombination davon besteht, ausgewählt ist mit Diathermie in kapazitiver Betriebsweise appliziert wird.

7. Verfahren nach einem der vorhergehenden Ansprüche, wobei die Diathermie bei einer Frequenz zwischen 0,5 MHz und 0,8 MHz appliziert wird.

## Revendications

1. Procédé de traitement cosmétique de la peau comprenant l'application sur la peau affectée d'une diathermie conjointement avec un gel conducteur et une ou plusieurs huiles et/ou une autre substance choisie dans le groupe constitué de l'acide hyaluronique, du beurre de karité, de l'aloe vera et des vitamines, ou d'une combinaison de ceux-ci, dans lequel le gel conducteur est appliqué avec une diathermie en mode résistif et l'huile ou les huiles sont appliquées avec une diathermie en mode capacitif.

2. Procédé selon la revendication 1, dans lequel ladite huile est choisie dans le groupe constitué de l'huile d'amande, de l'huile d'argan, de l'huile de marron d'Inde, de l'huile d'eucalyptus, de l'huile de gaulthérie naturelle, de l'huile de cynorhodon, de l'huile de carotte, de l'huile de camphre, des huiles de raffermissement, des huiles anticellulite et des huiles lipolytiques, ou d'une combinaison de celles-ci.

3. Procédé selon la revendication 2, dans lequel ladite huile est l'huile d'amande, l'huile d'argan, l'huile de marron d'Inde, l'huile d'eucalyptus, l'huile de gaulthérie naturelle, l'huile de cynorrhodon, l'huile de carotte, l'huile de camphre, ou une combinaison de celles-ci.

4. Procédé selon l'une quelconque des revendications précédentes, dans lequel la diathermie est appliquée entre 20 minutes et 1 heure et demie et à raison d'une fois par jour.

5. Procédé selon l'une quelconque des revendications précédentes, dans lequel la diathermie est appliquée la moitié du temps en mode capacitif et la moitié du temps en mode résistif.

6. Procédé selon l'une quelconque des revendications précédentes, dans lequel ladite autre substance choisie dans le groupe constitué de l'acide hyaluronique, du beurre de karité, de l'aloe vera et des vitamines, ou d'une combinaison de ceux-ci, est appliquée avec une diathermie en mode capacitif.

7. Procédé selon l'une quelconque des revendications précédentes, dans lequel la diathermie est appliquée à une fréquence entre 0,5 MHz et 0,8 MHz.
